# EUROPEAN PATENT APPLICATION

(11) **EP 3 372 610 A1**
(43) Date of publication of application: **12.09.2018**
(21) Application number: 17159776.8
(22) Date of filing: 08.03.2017
(51) Int. Cl.: C07F 9/24, C07H 21/00

(54) **NOVEL PHOSPHORYLATION REAGENTS AND USES THEREOF**

(71) Applicant: ETH Zurich, 8092 Zurich (CH)
(72) Inventor: HALL, Jonathan, 4143 Dornach (CH); PRADERE, Ugo, 8049 Zürich (CH)

(57) **Abstract**

The present invention relates to compounds of formula **(I)** 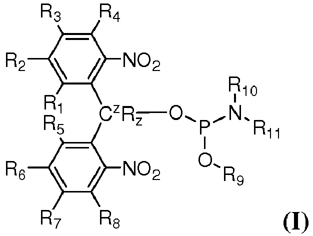 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-; or independently thereof
R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy, wherein preferably R_{z} is H;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl;
and the use of the compounds in a method of converting terminal hydroxyl groups of oligonucleotides into phosphate monoesters.

## Description

The present invention relates to the field of nucleotide chemistry. More specifically, the invention relates to the field of oligonucleotide synthesis including the chemical modification of oligonucleotides. In particular, the present invention provides for novel compounds of formula (I) and their use for converting terminal hydroxyl groups of oligonucleotides, preferably of converting terminal 5'-hydroxyl groups of oligonucleotides, into phosphate monoesters.

### RELATED ART

The current state of the art in oligonucleotide synthesis is automated solid phase synthesis using phosphoramidite chemistry (McBride, et al., Tetrahedron Letters (1983) 24, 245-248; Sinha, et al., Tetrahedron Letters (1983) 24, 5843-5846). Phosphoramidite chemistry, together with related methods such as hydrogen phosphonate chemistry, has been extensively reviewed with respect to their uses in oligonucleotide chemistry (Beaucage S.L., Iyer R.P., Tetrahedron (1992) 48, 2223-2311; Beaucage S.L., Iyer R.P., Tetrahedron (1993) 49, 1925-1963). During solid phase oligonucleotide synthesis, a series of nucleotide monomers are sequentially attached, via their phosphoramidite derivatives, in a predetermined order to either, depending on the direction of chain extension, the 5'-functional group or the 3'-functional group of the growing oligonucleotide strand. The oligonucleotide strand is anchored to an insoluble moiety such as controlled pore glass or polystyrene resin beads.

The method of attachment of each monomer is generally comprised of the following steps: (1) Deprotection of the reactive functionality. The common reactive functionality is the 5'-hydroxyl group of the terminal nucleoside. This functionality is usually protected with a 4,4'-dimethoxytrityl (DMT) moiety that can be removed via acid treatment. (2) Coupling by addition of a phosphoramidite derivative and an activator. The phosphoramidite derivative is usually a nucleoside phosphoramidite, however, it may also be a phosphoramidite derivatized with a different organic moiety. (3) Capping of unreacted terminal functional groups. This step introduces an inert protective group that prevents further coupling to failure sequences. (4) Oxidation of the newly formed phosphorous nucleotide backbone linkage from the trivalent phosphite to the stable pentavalent phosphate state. (5) After a washing step, the process is repeated.

In further detail and by way of example, during the deprotection step the trityl group attached to the 5'-carbon of the pentose sugar of the recipient nucleotide is removed by trichloroacetic acid (TCA) or dichloroacetic acid (DCA) in a suitable solvent such as dichloromethane or toluene, leaving a reactive hydroxyl group. The next phosphoramidite monomer is added in the coupling step. An activator such as tetrazole, a weak acid, is used to react with the coupling nucleoside phosphoramidite, forming a tetrazolyl phosphoramidite intermediate. This intermediate then reacts with the hydroxyl group of the recipient and the 5' to 3' linkage is formed. The tetrazole is reconstituted and the process continues. A coupling failure results in an oligonucleotide still having a reactive hydroxyl group on the 5'-end. To prevent these oligonucleotides from remaining reactive for the next cycle (which would produce an oligonucleotide with a missing nucleotide), they are removed from further synthesis by being irreversibly capped by an acetylating reagent such as a mixture of acetic anhydride and N-methylimidazole. This reagent reacts only with the free hydroxyl groups to cap the oligonucleotides. In the next step, the phosphite linkage between the growing oligonucleotide and the most recently added nucleotide is oxidized by reaction with iodine as a mild oxidant in tetrahydrofuran (THF) and water. The water acts as the oxygen donor and the iodine forms an adduct with the phosphorous linkage. The adduct is decomposed by the water leaving a stable phosphotriester linkage. Alternatively, the phosphite is converted to phosphorothioate using one of several possible sulfurizing reagents commonly employed in oligonucleotide synthesis. These include 3H-1,2-benzodithiol-3-one 1,1-dioxide, 3-((dimethylamino-methylidene)amino)-3H-1,2,4-dithiazaoline-3-thione and phenylacetyl disulphide (Iyer, R.P. J. Am. Chem. Soc. (1990) 112, 1253-1254).

The addition of a 5'-monophosphate group to oligonucleotides such as oligodeoxyribonucleotides (DNA) and oligoribonucleotides (RNA) is a key modification which permits access to various biochemical reagents and products. For example, two oligonucleotides can be ligated enzymatically when one of the oligonucleotides is 5'-monophosphorylated and the other bears a natural 3'-hydroxyl group (Shuman S., J Biol Chem. (2009) 284, 17365-17369; Romaniuk P.J., Uhlenbeck O.C., Methods Enzymol. (1983) 100, 52-59). This procedure is extensively used in cloning and gene construction for assembly of long DNA oligonucleotides. A second example involves RNA interference (RNAi), which involves a double stranded small interfering RNA (siRNA) that triggers the cleavage of target messenger RNA (mRNA) by association of the intracellularly-phosphorylated siRNA guide strand with the RISC endonuclease Argonaute 2 and the target mRNA (Ago2) (Fire A., et al., Nature (1998) 391, 806-811; Nykanen A., et al., Cell (2001) 107, 309-321; Meister G., et al., Mol. Cell (2004) 15, 185-197). The intracellular phosphorylation of unmodified siRNAs is known to be rather efficient (Kenski D.M., et al., Nucleic Acids Res. (2009) 38, 660-671; Kenski D.M., et al. Nucleic Acid Ther. (2012) 22, 90-95), however for siRNAs to be used in animals or humans (*in vivo*), chemically-modified RNAs are required. Chemically modified siRNAs are, however, less efficiently phosphorylated by intracellular kinases (e.g. hClp 1 kinase (Weitzer S., Martinez J., Nature (2007) 447, 222-226) and thus it may be advantageous to bypass this step by providing siRNAs in which the guide strand is pre-phosphorylated during the solid-phase chemical synthesis of the RNA (Fisher M., et al., Nucleic Acids Res. (2007), 35, 1064-1074; Watts J.K., et al., Nucleic Acids Res. (2007) 35, 1441-1451). Single-stranded siRNA (ss-siRNA) have been reported to maintain the ability of activating the RNAi pathway (Lima W.F., et al., Cell (2012) 150, 883-894). Those molecules have advantageous properties compared to double stranded siRNAs which includes efficient tissue distribution. The phosphorylation of ss-siRNA has been identified as a critical determinant for their activity (Haringsma H.J., et al., Nucleic Acids Res. (2012) 40, 4125-4136; Lima W.F., et al., Cell (2012) 150, 883-894), and this also includes the use of phosphate mimics.

The 5'-monophosphorylation of oligonucleotides is generally conducted enzymatically on the unmodified oligonucleotide at its terminal 5'-hydroxyl (Chaconas G., van de Sande J.H., Methods Enzymol. (1980) 65, 75-85). A kinase transfers a monophosphate moiety from adenosine triphosphate (ATP) to the free 5'-hydroxyl of the oligonucleotide. This process is easily performed on a small scale, however is tedious to scale-up. Chemical synthesis leading to 5'-phosphate oligonucleotides has also been described involving the use of a commercially-available phosphoramidite **1**, a dimethoxytrityl (DMT)-substituted phosphoramidite derivative of sulfonyldiethanol (Nagata S., et al., Nucleic Acid Res. (2010) 38, 7845-7857). This phosphoramidite is coupled to the terminal 5'-hydroxyl of a support-bound oligonucleotide in the final coupling step under routine conditions. Conventional cleavage of the oligonucleotide from the solid support and removal of protecting groups on the nucleobases and the phosphate sugar backbone is carried out by treatment with aqueous ammonia. However, in the process described by Nagata et al and using the phosphoramidite **1**, deprotonation of the acidic proton on the carbon atom in the alpha position to the sulfonyl group of the terminal phosphate group also occurs leading to the loss of the DMT group by a ß-elimination process (Nagata S., et al., Nucleic Acid Res. (2010) 38, 7845-7857). This is inconvenient because an important function of the DMT lipophilic group is to enable an efficient separation by reverse-phase high-performance liquid chromatography (RP-HPLC) or lipophilic affinity cartridge of the *bona fide* oligonucleotide product (full-length, N nucleotides) from small amounts of contaminating "failure sequences", i.e. oligonucleotide sequences of lengths N-1, N-2, etc. which are inevitably obtained during conventional oligonucleotide solid-phase synthesis. Use of this kind of masked phosphoramidites to introduce the terminal phosphate therefore is limited to short oligonucleotides, e.g. up to approximately 20 nucleotides in length, and produces products of moderate purity.

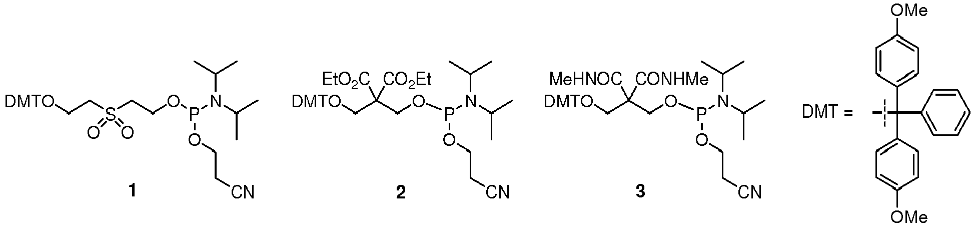

The use of commercially-available phosphoramidites 2 and 3 is compatible with purification of the DMT-protected oligonucleotide (Guzaev, A., et al. Tetrahedron (1995) 51, 9375-9384). In these cases, the DMT group is cleaved under conventional acidic conditions, but the release of the final 5'-phosphorylated oligonucleotide requires an additional treatment under basic conditions. This is compatible with synthesis of DNAs, however RNA is unstable in basic conditions (Li Y., Breaker R.R., J. Am. Chem. Soc. (1999) 121, 5364-5372) and therefore the method is incompatible with RNA synthesis.

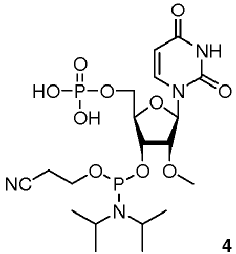

As an alternative to these procedures, a series of 5'-phosphate nucleoside 3'-phosphoramidites were introduced which can be coupled as the last nucleotide of the oligonucleotide sequence (e.g. uridine phosphoramidite **4**) (Lima W.F., et al., Cell (2012) 150, 883-894). This strategy requires the synthesis and use of four 5'-phosphate nucleoside 3'-phosphoramidites (or more if modifications are included) to be able to include the four different nucleosides into RNA. Furthermore, oligonucleotides synthesized using this approach do not have a lipophilic group such as DMT to facilitate purification, either by reverse-phase HPLC, ion exchange HPLC or lipophilic affinity cartridge.

### SUMMARY OF THE INVENTION

This present invention provides compounds of formula **(I)** and their use in a method of converting terminal hydroxyl groups of oligonucleotides, preferably of converting terminal 5'-hydroxyl groups of oligonucleotides, into phosphate monoesters. Preferably, the present invention provides compounds of formula **(I)** and their use for the solid-phase synthesis of 5'-phosphorylated oligonucleotides including DNA, RNA and modified equivalents and variants.

Thus, in a first aspect, the present invention provides for a compound of formula **(I)** 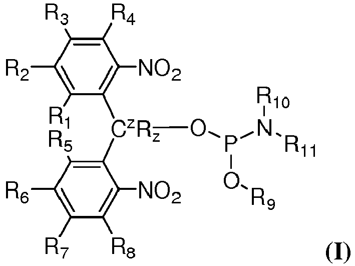 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-, and thus connecting the two aryl groups of said DNB moiety; or independently thereof
R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³-independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl, and thus forming an optionally substituted fused saturated heterocyclic ring, an optionally substituted fused partly unsaturated heterocyclic ring or an optionally substituted fused hetero-aromatic ring;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy, wherein preferably R_{z} is H;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl; and the carbon atom C^{Z} represents a carbon atom of said 4,4'-dinitrobenzhydryl-moiety (DNB-moiety) of said compound of formula (I). The superscript "z" is added for definition purpose since in a very preferred embodiment of the present invention, the selection of R₁, R₂, R₃, R₄, R₅, R₆, R₇, R₈ and R_{z} together is such that the carbon atom C^{Z} is not an asymmetric carbon atom and, thus, does not become a chiral carbon atom.

In a further aspect, the present invention provides for a method of converting terminal hydroxyl groups of oligonucleotides, preferably of converting terminal 5'-hydroxyl groups of oligonucleotides, into phosphate monoesters. Thus, in said aspect, the present invention provides for a method of converting a terminal hydroxyl group of an oligonucleotide, preferably of converting a terminal 5'-hydroxyl group of an oligonucleotide, into a phosphate monoester, comprising of the steps of (a) reacting a compound of formula **(I)** with said terminal hydroxyl group of an oligonucleotide, 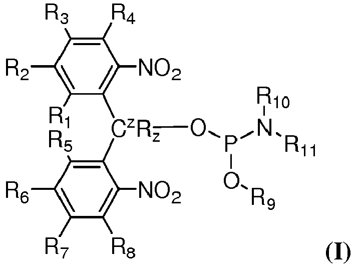 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-, and thus connecting the two aryl groups of said DNB moiety; or independently thereof
R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³-independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl, and thus forming an optionally substituted fused saturated heterocyclic ring, an optionally substituted fused partly unsaturated heterocyclic ring or an optionally substituted fused hetero-aromatic ring;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy, wherein preferably R_{z} is H;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl; and the carbon atom C^{Z} represents a carbon atom of said 4,4'-dinitrobenzhydryl-moiety (DNB-moiety) of said compound of formula **(I)**; and wherein the -NR₁₀R₁₁ moiety of the compound of formula **(I)** is removed; (b) oxidizing the P atom of compound **(I)** of the resulting product of a) and removing said R₉ residue to generate a phosphate diester of said oligonucleotide comprising said DNB-moiety as one ester group; (c) purifying said phosphate diester of said oligonucleotide comprising said DNB-moiety of b); and (d) removing said DNB-moiety comprised by said phosphate diester of said oligonucleotide to generate said phosphate monoester of said oligonucleotide.

The novel phosphoramidites of formula **(I)** of the present invention can be coupled to the terminal hydroxyl group of oligonucleotides, preferably to the terminal 5'-hydroxyl of oligonucleotides on solid support under conventional conditions known to the person skilled in the art and are, thus, fully compatible with standard procedures for solid phase oligonucleotide synthesis. Classes of oligonucleotides to which it can be coupled include DNA, RNA or chemically-modified variants thereof. Advantageously, conventional cleavage of the oligonucleotide under basic conditions from the solid support does not result in loss of the lipophilic substituent comprising the 4,4'-dinitrobenzhydryl (DNB)-moiety. Moreover, after purification, preferably HPLC-purification, further preferably reverse phase or ion-exchange HPLC-purification, of the 5'-phosphorylated oligonucleotide, said DNB-moiety can be easily removed by simple UV irradiation, typically and preferably for 5 to 10 min at 365 nm, yielding the free 5'- phosphorylated oligonucleotide. The DNB-moiety, in particular the DNB-moiety wherein R₁, R₂, R₃, R₄, R₅, R₆, R₇, and R₈ represent hydrogen, is highly sensitive to irradiation as compared to other substituted 2-nitrobenzyl groups. Moreover, short irradiation at this wavelength does not degrade the oligonucleotide (Kladwang W., et al., Sci Rep. (2012) 2, 517).

The preparation and use of the novel phosphoramidites of formula **(I)** of the present invention introduce an efficient and cheap access to phosphorylated oligonucleotides, in particular to 5'-phosphorylated oligonucleotides, and is thus advantageous compared to known procedures.

Further aspects and embodiments of the present invention will become apparent as this description continues.

### DETAILED DESCRIPTION OF THE INVENTION

Unless defined otherwise, all technical and scientific terms used herein have the same meanings as commonly understood by one of ordinary skill in the art to which this invention belongs.

Each "alkyl" moiety either alone or as part of a larger group such as alkoxy or alkylene is a straight or branched carbon chain and is preferably C₁-C₉alkyl, more preferably C₁-C₃alkyl. Examples include methyl, ethyl, *n*-propyl, prop-2-yl (*iso*-propyl), *n*-butyl, tert-butyl, but-2-yl, 2-methyl-prop-1-yl or 2-methyl-prop-2-yl. Examples of an alkoxy include methoxy, ethoxy, propoxy, *iso*-propoxy, *n*-butoxy, *sec*-butoxy, *tert*-butoxy, *n*-pentoxy, *neo*-pentoxy, *n*-hexoxy. As described herein, alkoxy may include further substituents such as halogen atoms leading to haloalkoxy moieties.

Each haloalkyl moiety either alone or as part of a larger group such as haloalkoxy is an alkyl group substituted by one or more of the same or different halogen atoms. Examples include difluoromethyl, trifluoromethyl, chlorodifluoromethyl and 2,2,2-trifluoro-ethyl. Haloalkyl moieties include for example 1 to 5 halo substituents, or 1 to 3 halo substituents.

Each "alkylene" moiety is a straight or branched carbon chain and is, for example, -CH₂-, -CH₂-CH₂-, -CH(CH₃)-, -CH₂-CH₂-CH₂-, -CH(CH₃)-CH₂- or -CH(CH₂CH₃)-.

The term "alkenyl" is defined identically as "alkyl," except for containing a carbon-carbon double bond. The term "alkenylene" is defined identically to "alkylene" except for containing a carbon-carbon double bond. The term "alkdienylene" is defined identically as "alkenylene" except the group contains two carbon-carbon double bonds, either conjugated or non-conjugated.

Each alkenyl moiety either alone or as part of a larger group such as alkenyloxy or alkenylene is a straight or branched carbon chain and is preferably C₂-C₆alkenyl, more preferably C₂-C₄alkenyl. Each moiety can be of either the (*E*)- or (*Z*)-configuration. Examples include vinyl and allyl. A compound of the present invention comprising an alkenyl moiety thus may include either said compound with said alkenyl moiety in its (*E*)-configuration, said compound with said alkenyl moiety in its (*Z*)-configuration and mixtures thereof in any ratio.

Each "cycloalkyl" moiety can be in mono- or bi-cyclic form, typically and preferably in monocyclic form, and preferably contains 3 to 8 carbon atoms, more preferably 3 to 7 carbon atoms. Examples of monocyclic cycloalkyl groups include cyclopropyl, cyclobutyl and cyclohexyl.

Halogen is fluorine, chlorine, bromine or iodine.

The term "heterocyclic ring" refers to a saturated or partially unsaturated carbocyclic ring containing one to three heteroatoms selected from nitrogen, oxygen and sulfur as ring members. The term "heterocyclic ring" preferably refers to a 5-7-membered saturated or partially unsaturated carbocyclic ring containing one to three heteroatoms selected from nitrogen, oxygen and sulfur as ring members. Such rings do not contain adjacent oxygen atoms, adjacent sulfur atoms, or adjacent oxygen and sulfur atoms within the ring. Examples include aziridine, azetidine, pyrrolidine, pyrazolidine, imidazolidine, piperidine, piperazine, dioxane, dioxolane, tetrahydrofurane, morpholine, oxazolidine and isooxazolidine. Preferred examples are pyrrolidine, pyrazolidine, imidazolidine, piperidine, piperazine, dioxane, dioxolane, tetrahydrofurane, morpholine, oxazolidine and isooxazolidine.

The term "heteroaromatic ring" as used herein, refers to an aromatic ring system containing at least one heteroatom, and preferably up to three heteroatoms selected from nitrogen, oxygen and sulfur as ring members. The term "heteroaromatic ring" preferably refers to a 5-7-membered aromatic ring system containing at least one heteroatom, and preferably up to three heteroatoms selected from nitrogen, oxygen and sulfur as ring members. Heteroaromatic rings do not contain adjacent oxygen atoms, adjacent sulfur atoms, or adjacent oxygen and sulfur atoms within the ring. Preferred examples are include pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl, and further preferred are pyridine, pyrrolidine, piperidine, piperazine, morpholine, pyrrole, and imidazole.

Where a group is said to be optionally substituted, preferably there are optionally 1-3 substituents, more preferably optionally 1-2 substituents. Where a group is said to be optionally substituted, and where there are more than one substituents for said optional substitution of said group, said more than one substituents can either be the same or different.

The term "4,4'-dinitrobenzhydryl-moiety" or "4,4'-dinitrobenzhydryl (DNB)-moiety" or "DNB-moiety", as interchangeably used herein, refers to the moiety depicted below which is part of the compounds of formula (I) of the present invention; 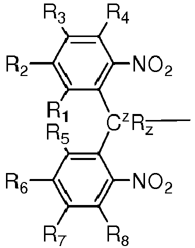 wherein carbon atom C^{Z} represents a carbon atom of said DNB-moiety and the superscript "z" is added for definition purpose since in a very preferred embodiment of the present invention, the selection of R₁, R₂, R₃, R₄, R₅, R₆, R₇, R₈ and R_{z} together is such that the carbon atom C^{Z} is not an asymmetric carbon atom and, thus, does not become a chiral carbon atom.

The term "oligonucleotide", as used herein, refers to a nucleic acid sequence comprising 2 or more nucleotides, for example up to several hundred or more, preferably at least 2 nucleotides to 300 nucleotides. Oligonucleotides are polyribonucleotides or polydeoxyribonucleotides and are preferably selected from unmodified RNA, unmodified DNA, modified RNA or modified DNA. The modification may comprise the backbone and/or the nucleotide analogues as further described herein. Oligonucleotides are preferably selected from single-stranded DNA, double-stranded DNA, single-stranded RNA or double-stranded DNA, further preferably from single-stranded. The term "modified oligonucleotide", such as modified RNA or modified DNA, as used herein, refers to an oligonucleotide as defined above and having at least one modified internucleoside linkage and/or at least one sugar modification and/or at least one base modification compared to a naturally occurring ribonucleotide- or deoxyribonucleotide-based oligonucleotide. A "modified internucleoside linkage" indicates the presence of a modified version of the phosphodiester which does not occur naturally in RNA and DNA. Examples of internucleoside linkage modifications, which are known to the skilled person in the art and which are compatible with the present invention, are and include in particular, phosphoramidate, phosphorodiamidate, phosphorothioate, phosphorodithioate, H-phosphonate, methyl phosphonate and methyl phosphonothioate. A "sugar modification" indicates the presence of a modified version of the ribosyl moiety as naturally occurring in RNA and DNA (i.e. the furanosyl moiety), such as bicyclic sugars, tetrahydropyrans, morpholinos, 2'-modified sugars, 3'-modified sugars, 4'-modified sugars, 5'-modified sugars, and 4'-subsituted sugars. Examples of suitable sugar modifications are known to the skilled person in the art and include, but are not limited to, 2'-*O*-modified RNA nucleotide residues, such as 2'-*O*-alkyl or 2'-*O*-(substituted)alkyl e.g. 2'-*O*-methyl, 2'-*O*-(2-cyanoethyl), 2'-*O*-(2-methoxy)ethyl (2'-MOE), 2'-*O*-(2-thiomethyl)ethyl; 2'-*O*-(haloalkoxy)methyl e.g. 2'-*O*-(2-chloroethoxy)methyl (MCEM), 2'-0-(2,2-dichloroethoxy)methyl (DCEM); 2'-*O*-alkoxycarbonyl e.g. 2'-*O*-[2-(methoxycarbonyl)ethyl] (MOCE), 2'-*O*-[2-(*N*-methylcarbamoyl)ethyl] (MCE), 2'-*O*-[2-(*N,N*-dimethylcarbamoyl)ethyl] (DMCE), in particular a 2'-*O*-methyl modification or a 2'-*O*-methoxyethyl (2'-*O*-MOE). Another important modification includes "bridged" or "bicylic" nucleic acid (BNA) modified sugar moieties, such as found in e.g. locked nucleic acid (LNA), *xylo*-LNA, α-L-LNA, β-D-LNA, cEt (2'-0,4'-C constrained ethyl) LNA, cMOEt (2'-*O*,4'-*C* constrained methoxyethyl) LNA, ethylene-bridged nucleic acid (ENA), hexitol nucleic acid (HNA), fluorinated HNA (F-HNA), pyranosyl-RNA (p-RNA), 3'-deoxypyranosyl-DNA (p-DNA); or other modified sugar moieties, such as morpholino (PMO), cationic morpholino (PMOPlus) or PMO-X, all known to the skilled person in the art. The term "base modification", as used herein refers to the modification of a naturally occurring base in RNA and/or DNA (i.e. pyrimidine or purine base). A base modification is known to the skilled person in the art and includes, but is not limited to, a modified version of the natural purine and pyrimidine bases (e.g. adenine, uracil, guanine, cytosine, and thymine), such as hypoxanthine, pseudouracil, pseudothymine, 2-thiopyrimidine (e.g. 2-thiouracil, 2-thiothymine), 2,6-diaminopurine, 5-substituted pyrimidine (e.g. 5-halouracil, 5-methyluracil, 5-methylcytosine) 7-deazaguanine, 7-deazaadenine, 7-aza-2,6-diaminopurine, 8-aza-7-deazaguanine, 8-aza-7-deazaadenine, or 8-aza-7-deaza-2,6-diaminopurine. It is also encompassed by the invention that said oligonucleotide comprises more than one, the same or different, internucleoside linkage modification, sugar modification and/or base modification. Thus, oligonucleotides, as referred to in this invention can consist of any combinations of the nucleotides and their modifications described above and can have either a few, e.g. up to 20, or many, e.g. 20 to several hundred or more, nucleotides incorporated in their chain. Preferably, oligonucleotides of the present invention comprise at least 2 nucleotides to 300 nucleotides or their modifications described above.

The term "terminal hydroxyl group", as used herein, refers to a hydroxyl group of either the 3'-end nucleotide unit of an oligonucleotide or the 5'-end nucleotide unit of an oligonucleotide, or a hydroxyl group of a spacer or a modifier linked to either the 3'-end nucleotide unit of an oligonucleotide or the 5'-end nucleotide unit of an oligonucleotide. Examples of terminal hydroxyl groups include, but are not limited to the 3'-hydroxyl group of the terminal nucleotide unit, in the case in which the oligonucleotide is synthesized in a 5'- to 3'-direction, or the 5'-hydroxyl group of the terminal nucleotide unit, in the case in which the oligonucleotide synthesis is conducted in a 3'- to 5'-direction.

Thus, in a first aspect, the present invention provides for a compound of formula (I) 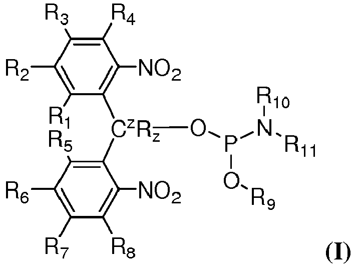 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-, and thus connecting the two aryl groups of said DNB moiety; or independently thereof
R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³-independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl, and thus forming an optionally substituted fused saturated heterocyclic ring, an optionally substituted fused partly unsaturated heterocyclic ring or an optionally substituted fused hetero-aromatic ring;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy, wherein preferably R_{z} is H;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl; and the carbon atom C^{Z} represents a carbon atom of said 4,4'-dinitrobenzhydryl-moiety (DNB-moiety) of said compound of formula (I). The superscript "z" is added for definition purpose since in a very preferred embodiment of the present invention, the selection of R₁, R₂, R₃, R₄, R₅, R₆, R₇, R₈ and R_{z} together is such that the carbon atom C^{Z} is not an asymmetric carbon atom and, thus, does not become a chiral carbon atom.

In a preferred embodiment of the present invention, said R_{z} is H.

In a preferred embodiment of the present invention, said 4,4'-dinitrobenzhydryl-moiety (DNB-moiety) of said compound of formula (I) is selected from 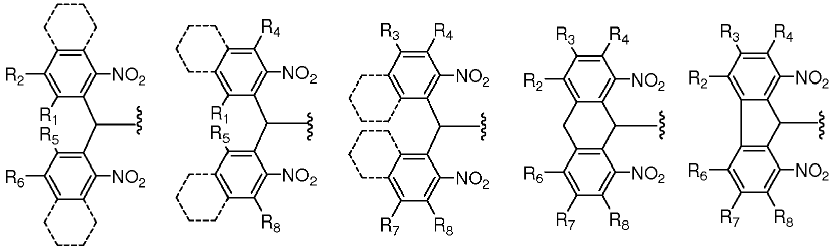 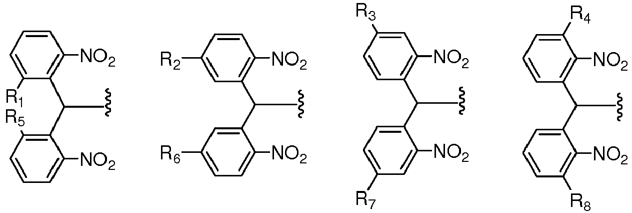 wherein R₁, R₂, R₃, R₄, R₅, R₆, R₇, and R₈ are independently of each other hydrogen, fluorine, chlorine, bromine, nitro, cyano, C₁-C₄alkyl, C₁-C₂alkoxy, C₁-C₂haloalkyl, C₁-C₂haloalkoxy; and wherein the dotted lines represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein the dotted lines represent independently of each other said bivalent residue -R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl; and wherein the wavy line indicates the attachment to the oxygen in said compound of formula (I). In a further preferred embodiment, said dotted lines in said formula represent independently of each other said bivalent residue -R¹²R¹³-, wherein each of said bivalent residue -R¹²R¹³- independently at each occurrence is C₃-C₄alkylene, C₃-C₄alkenylene or C₃-C₄alkdienylene, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 5-6-membered heterocyclic ring or a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-6-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene or C₄alkdienylene, and thus forming a cyclopentyl, cyclohexyl or phenyl, each independently optionally substituted with halogen, nitro, cyano, C₁-C₂alkyl C₁-C₂alkoxy, C₁-C₂haloalkyl; or independently thereof, said bivalent residue -R¹²R¹³-independently at each occurrence together with the vicinal C atoms to which they are attached independently of each other form a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered hetero-aromatic ring is independently at each occurrence selected from pyridine, pyrazine, or imidazole.

In a further preferred embodiment of the present invention, said 4,4'-dinitrobenzhydryl-moiety (DNB-moiety) of said compound of formula (I) is selected from 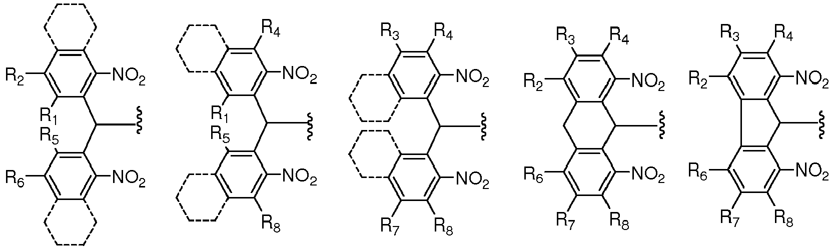 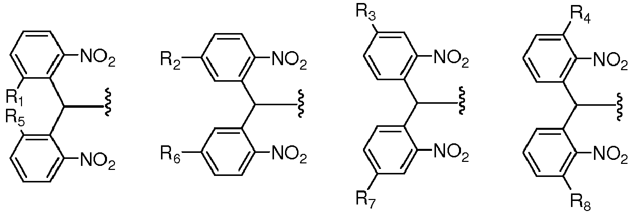 wherein R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃; and wherein the dotted lines represent independently of each other said bivalent residue -R¹²R¹³- wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein the dotted lines represent independently of each other said bivalent residue -R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl; wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine; and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl; and wherein the wavy line indicates the attachment to the oxygen in said compound of formula (I). In a further preferred embodiment, said dotted lines in said formula represent independently of each other said bivalent residue -R¹²R¹³-, wherein each of said bivalent residue -R¹²R¹³- independently at each occurrence is C₃-C₄alkylene, C₃-C₄alkenylene or C₃-C₄alkdienylene, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 5-6-membered heterocyclic ring or a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-6-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene or C₄alkdienylene, and thus forming a cyclopentyl, cyclohexyl or phenyl, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 6-membered hetero-aromatic ring, each independently optionally substituted with halogen, nitro, cyano, C₁-C₂alkyl C₁-C₂alkoxy, C₁-C₂haloalkyl, wherein said 5-6-membered hetero-aromatic ring is independently at each occurrence selected from pyridine, pyrazine, or imidazole.

In a further preferred embodiment of the present invention, said 4,4'-dinitrobenzhydryl-moiety (DNB-moiety) of said compound of formula (I) is selected from 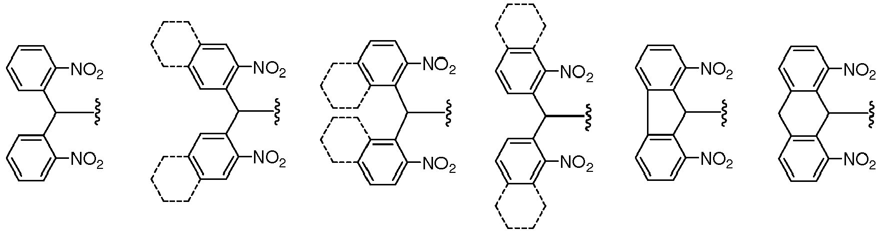 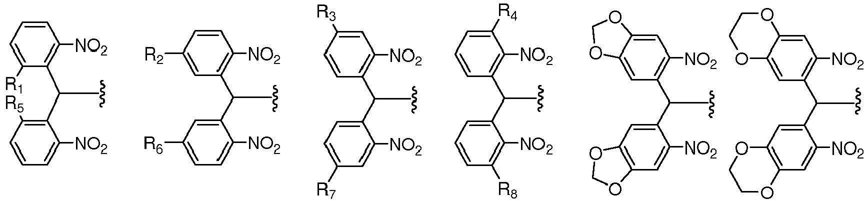 wherein R₁, R₂, R₃, R₄, R₅, R₆, R₇, and R₈ are independently of each other hydrogen, fluorine, chlorine, bromine, nitro, cyano, C₁-C₄alkyl, C₁-C₂alkoxy, C₁-C₂haloalkyl, C₁-C₂haloalkoxy; and wherein the dotted lines represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl (and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein the dotted lines represent independently of each other said bivalent residue -R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl; and wherein the wavy line indicates the attachment to the oxygen in said compound of formula (I). In a further preferred embodiment, said dotted lines in said formula represent independently of each other said bivalent residue -R¹²R¹³-, wherein each of said bivalent residue -R¹²R¹³- independently at each occurrence is C₃-C₄alkylene, C₃-C₄alkenylene or C₃-C₄alkdienylene, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 5-6-membered heterocyclic ring or a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-6-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene or C₄alkdienylene, and thus forming a cyclopentyl, cyclohexyl or phenyl, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 6-membered hetero-aromatic ring, each independently optionally substituted with halogen, nitro, cyano, C₁-C₂alkyl C₁-C₂alkoxy, C₁-C₂haloalkyl, wherein said 5-6-membered hetero-aromatic ring is independently at each occurrence selected from pyridine, pyrazine, or imidazole.

In a further preferred embodiment of the present invention, said 4,4'-dinitrobenzhydryl-moiety (DNB-moiety) of said compound of formula (I) is selected from 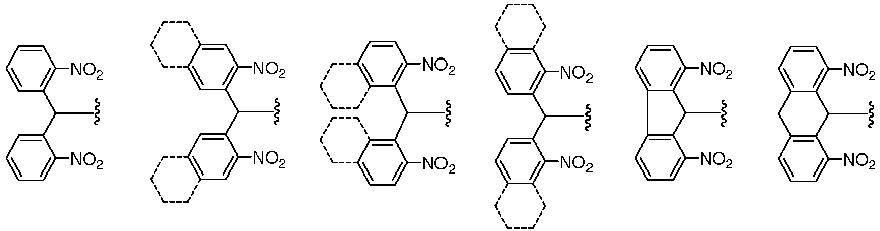 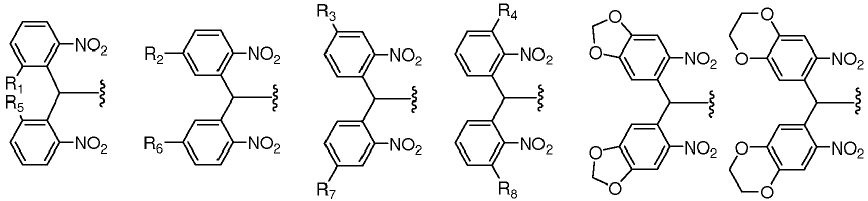 wherein said R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃; wherein the dotted lines in said formula represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue -R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl, wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl; and wherein the wavy line indicates the attachment to the oxygen in said compound of formula (I). In a further preferred embodiment, said dotted lines in said formula represent independently of each other said bivalent residue -R¹²R¹³-, wherein each of said bivalent residue -R¹²R¹³- independently at each occurrence is C₃-C₄alkylene, C₃-C₄alkenylene or C₃-C₄alkdienylene, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 5-6-membered heterocyclic ring or a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-6-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene or C₄alkdienylene, and thus forming a cyclopentyl, cyclohexyl or phenyl, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 6-membered hetero-aromatic ring, each independently optionally substituted with halogen, nitro, cyano, C₁-C₂alkyl C₁-C₂alkoxy, C₁-C₂haloalkyl, wherein said 5-6-membered hetero-aromatic ring is independently at each occurrence selected from pyridine, pyrazine, or imidazole.

In a further preferred embodiment of the present invention, R₉ is C₁-C₉alkyl optionally substituted with cyano, nitro, chlorine, fluorine, bromine, -NHC(O)C₁-C₃alkyl, -NHC(O)C₁-C₃haloalkyl; aryl, C₁-C₆alkylenearyl, C₁-C₆alkylenediaryl independently of each other optionally substituted with cyano, nitro, chlorine, fluorine, bromine, C₁-C₄alkoxy, C₁-C₄haloalkyl. In a further preferred embodiment, said R₉ is C₁-C₃alkyl optionally substituted with cyano, chlorine, fluorine and bromine; aryl, C₁-C₃alkylenearyl, C₁-C₃alkylenediaryl, independently of each other optionally substituted with cyano, nitro, chlorine, fluorine, bromine, C₁-C₂alkoxy, C₁haloalkyl. In a further preferred embodiment of the present invention, said R₉ is C₁-C₃alkyl, 2-cyanoethyl, 2,2,2-trichloroethyl, 2,2,2-tribromoethyl, -(CH₂)ₙNHC(O)CF₃ wherein n=3-6; phenyl, C₁-C₃alkylenephenyl, benzhydryl, independently of each other optionally substituted with cyano, nitro, chlorine, fluorine, bromine, C₁-C₂alkoxy, -CF₃. In an again further preferred embodiment of the present invention, said R₉ is methyl, ethyl, 2-cyanoethyl, 2,2,2-trichloroethyl, 2,2,2-tribromoethyl, -(CH₂)₅NHC(O)CF₃, phenyl, chlorophenyl preferably p-chlorophenyl, nitrophenyl preferably p-nitrophenyl, nitrophenylethyl preferably p-nitrophenylethyl, benzyl, ethoxyphenyl, methoxyphenyl, C₁-C₃alkylenephenyl or benzhydry, independently of each other optionally substituted with cyano, nitro, chlorine, fluorine, bromine, methoxy, -CF₃. In an again further preferred embodiment of the present invention, said R₉ is methyl, ethyl, 2-cyanoethyl, 2,2,2-trichloroethyl, 2,2,2-tribromoethyl, -(CH₂)₅NHC(O)CF₃, phenyl, chlorophenyl preferably p-chlorophenyl, nitrophenyl preferably p-nitrophenyl, nitrophenylethyl preferably p-nitrophenylethyl, benzyl, ethoxyphenyl, methoxyphenyl, C₁-C₃alkylenephenyl or benzhydryl independently of each other optionally mono-substituted with cyano, nitro, chlorine, fluorine, bromine, methoxy, -CF₃. In a very preferred embodiment of the present invention, said R₉ is methyl, ethyl, 2-cyanoethyl, or selected from 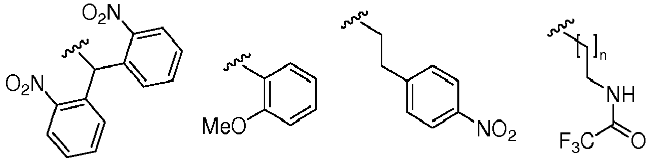 wherein the wavy line indicates the attachment to the oxygen in said compound of formula (I).

In a further very preferred embodiment of the present invention, said R₉ is 2-cyanoethyl or 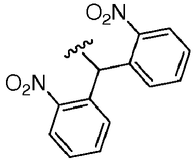 wherein the wavy line indicates the attachment to the oxygen in said compound of formula (I).

In a further very preferred embodiment of the present invention, said R₉ is 2-cyanoethyl.

In a preferred embodiment of the present invention, said R₁₀ and R₁₁ are independently C₁-C₃alkyl or together with the nitrogen atom to which they are attached form a heterocyclic ring, wherein said heterocyclic ring is selected from pyrollidine, piperidine, morpholine, wherein said heterocyclic ring is optionally substituted with C₁-C₃ alkyl, and wherein again further preferably said heterocyclic ring is optionally substituted with methyl. In a further very preferred embodiment, R₁₀ is equal to R₁₁. In a further very preferred embodiment said -NR₁₀R₁₁ is selected from

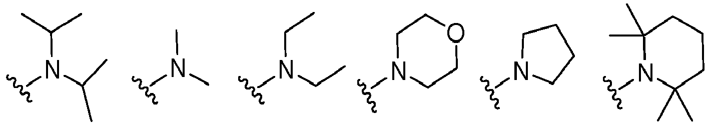

In a very preferred embodiment of the present invention, R₁₀ is equal to R₁₁ and R₁₀ and R₁₁ are iso-propyl or methyl.

In an again very preferred embodiment, the compound of formula (I) is selected from 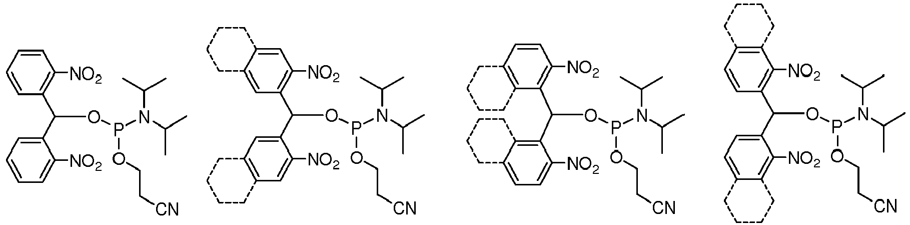 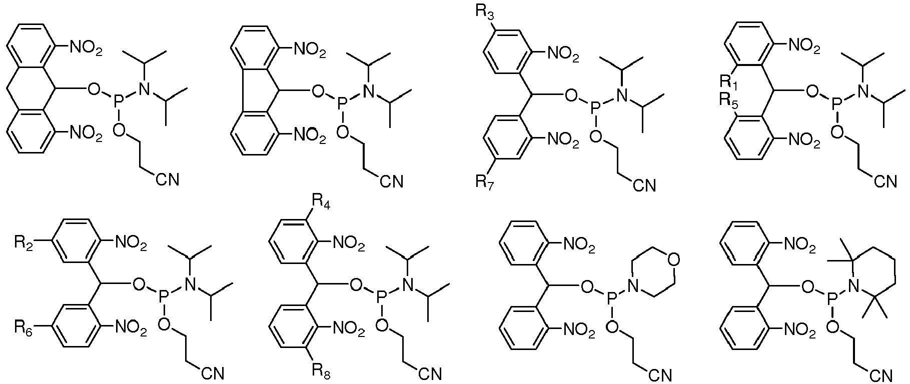 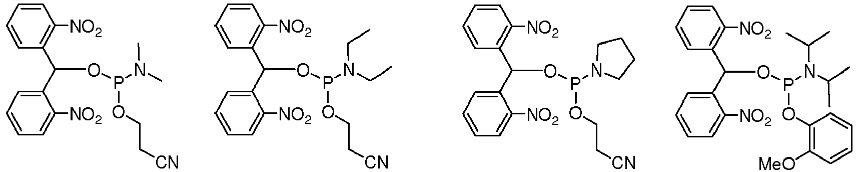 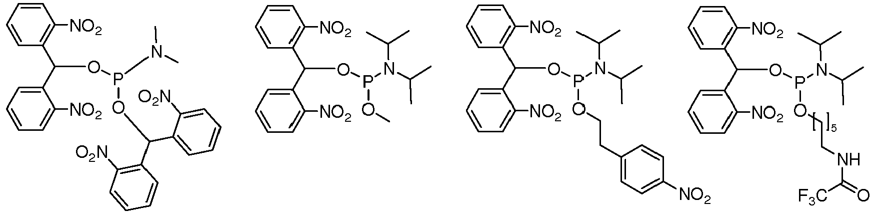 wherein said R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃; wherein the dotted lines in said formula represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue -R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl, wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene, C₃-C₄alkenylene or C₃-C₄alkdienylene, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 5-6-membered heterocyclic ring or a 5-6-membered hetero-aromatic ring, each independently optionally substituted with halogen, nitro, cyano, C₁-C₂alkyl C₁-C₂alkoxy, C₁-C₂haloalkyl, wherein said 5-6-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-6-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl. In a further preferred embodiment, said dotted lines in said formula represent independently of each other said bivalent residue -R¹²R¹³-, wherein each of said bivalent residue -R¹²R¹³- independently at each occurrence is C₃-C₄alkylene or C₄alkdienylene, and thus forming a cyclopentyl, cyclohexyl or phenyl, each independently optionally substituted with halogen, nitro, cyano, C₁-C₂alkyl C₁-C₂alkoxy, C₁-C₂haloalkyl; or independently thereof, said bivalent residue -R¹²R¹³- independently at each occurrence together with the vicinal C atoms to which they are attached independently of each other form a 6-membered hetero-aromatic ring, each independently optionally substituted with halogen, nitro, cyano, C₁-C₂alkyl C₁-C₂alkoxy, C₁-C₂haloalkyl, wherein said 5-6-membered hetero-aromatic ring is independently at each occurrence selected from pyridine, pyrazine, or imidazole.

In a very preferred embodiment, said R₁ is equal to R₅, said R₂ is equal to R₆, said R₃ is equal to R₇, or said R₄ is equal to R₈.

In a further very preferred embodiment, said R₁ is equal to R₅, said R₂ is equal to R₆, said R₃ is equal to R₇, and said R₄ is equal to R₈.

In a further very preferred embodiment, the selection of R₁, R₂, R₃, R₄, R₅, R₆, R₇, R₈ and R_{z} together is such that the carbon atom C^{Z} is not an asymmetric carbon atom and, thus, does not become a chiral carbon atom. The symmetrical lipophilic protecting group comprising said DNB-moiety, advantageously, allows not only for an efficient purification of the phosphorylated oligonucleotide, typically and preferably the 5'-phosphorylated oligonucleotide, product from failure sequences irrespective of oligonucleotide length, but furthermore has the added advantage to avoid the formation of diastereomeric mixtures at the terminal position. This further facilitates HPLC-purification process because only a single product peak in the HPLC chromatogram is produced.

In a further very preferred embodiment of the present invention, said compound is selected from the compound of formula **(Ia)**, **(Ib)**, **(Ic)** and **(Id)**

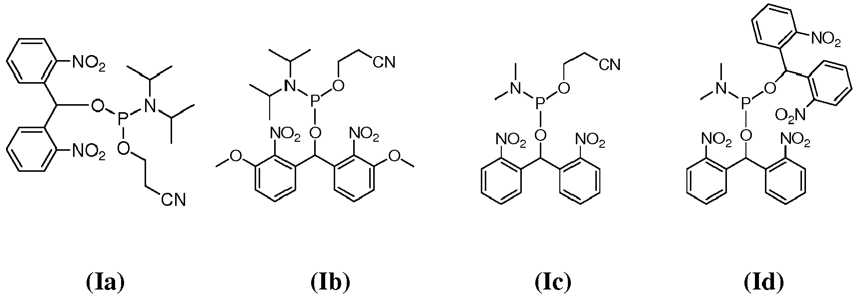

In a further very preferred embodiment of the present invention, said compound is the compound of formula **(Ia)**

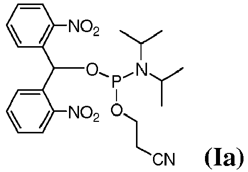

In a further aspect, the present invention provides for a method of converting terminal hydroxyl groups of oligonucleotides, preferably of converting terminal 5'-hydroxyl groups of oligonucleotides, into phosphate monoesters. Thus, in said aspect, the present invention provides for a method of converting a terminal hydroxyl group of an oligonucleotide, preferably of converting a terminal 5'-hydroxyl group of an oligonucleotide, into a phosphate monoester, comprising of the steps of (a) reacting a compound of formula **(I)** with said terminal hydroxyl group of an oligonucleotide, 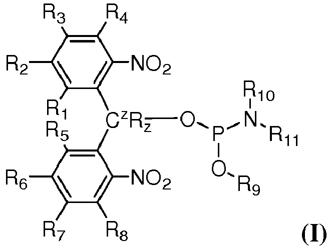 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-, and thus connecting the two aryl groups of said DNB moiety; or independently thereof
R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl, and thus forming an optionally substituted fused five-, six- or seven-membered saturated carbocyclic ring, an optionally substituted fused partly unsaturated five-, six- or seven-membered carbocyclic ring or an optionally substituted fused five-, six- or seven-membered aromatic ring; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl, and thus forming an optionally substituted fused saturated heterocyclic ring, an optionally substituted fused partly unsaturated heterocyclic ring or an optionally substituted fused hetero-aromatic ring;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy, wherein preferably R_{z} is H;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl; and the carbon atom C^{Z} represents a carbon atom of said 4,4'-dinitrobenzhydryl-moiety (DNB-moiety) of said compound of formula **(I)**; and wherein the -NR₁₀R₁₁ moiety of the compound of formula **(I)** is removed; (b) oxidizing the P atom of compound (I) of the resulting product of a) and removing said R₉ residue to generate a phosphate diester of said oligonucleotide comprising said DNB-moiety as one ester group; (c) purifying said phosphate diester of said oligonucleotide comprising said DNB-moiety of b); and (d) removing said DNB-moiety comprised by said phosphate diester of said oligonucleotide to generate said phosphate monoester of said oligonucleotide.

In further preferred embodiments of said method, said compound of formula (I) is defined as in any embodiment disclosed and described herein.

Thus, the present invention describes methods for the preparation of oligonucleotides with terminal phosphate moieties. The methods comprise the coupling of the novel phosphoramidite compound of formula (I) with terminal hydroxyl groups of oligonucleotides and the removal of the phosphate protective groups of compound (I) except the DNB-moiety of the compound (I), purifying the resulting diester comprising the 4,4'-dinitrobenzhydryl (DNB)-moiety and finally removing said DNB-moiety to provide oligonucleotides with a terminal phosphate.

The methods of the invention can be applied to the synthesis of oligonucleotides with either 5'-phosphate moieties, for cases in which the solid phase synthesis of the oligonucleotide is conducted in 3'- to 5'-direction, or with 3'-phosphate moieties, for cases in which the solid phase synthesis of the oligonucleotide is conducted in 5'- to 3'-direction.

The novel compounds of formula **(I)** and methods of the present invention are useful in any field that requires the chemical synthesis of oligonucleotides with terminal phosphate moieties, e.g. cloning, gene construction, ligase reactions, and post synthetic labeling of oligonucleotides.

The method of the invention requires that an unprotected hydroxyl group be reacted with the compound of formula **(I)** typically and preferably in the presence of an activator. There are several appropriate activators that are widely known to those skilled in the art of phosphoramidite chemistry, including, but not limited to 1H-tetrazole, 4,5-dicyanoimidazole, 5-ethylthiotetrazole, pyridinium trifluoracetate, benzimidazolium triflate, 5-benzylthiotetrazole and other activators as described in the literature and known to those skilled in the art.

Further, in the course of the method of converting terminal hydroxyl groups of oligonucleotides, preferably terminal 5'-hydroxyl groups of oligonucleotides, into phosphate monoesters in accordance with the present invention, the residue R₉ is removed typically and preferably after oxidation of the trivalent phosphor of the compound of formula **(I)** to a pentavalent phosphor. The oxidation as well as the removal of the residue R₉ are well-known to the skilled person in the art and has been described extensively (Beaucage S.L., Iyer R.P., Tetrahedron (1992) 48, 2223-2311; and references cited therein). Typically and preferably, the residue R₉ is the 2-cyanoethyl group, which is typically removed under mild basic conditions after oxidation to the pentavalent phosphate species.

Furthermore, the residue R₉ is typically and preferably removed concurrently when cleaving the linkage of the oligonucleotide to the solid support as known by the skilled person in the art. In a preferred embodiment, when R₉ is the 2-cyanoethyl group, then R₉ is removed concurrently by the typical and preferred final ammonium hydroxide treatment that is typically applied and required to cleave the linkage of the oligonucleotide to the solid support.

The cleaved oligonucleotide containing a terminal phosphate moiety, i.e. the phosphate diester of said oligonucleotide comprising said DNB-moiety may be recovered and isolated by a variety of known techniques including, but not limited to desalting, gel electrophoresis, anion exchange high pressure liquid chromatography (HPLC), reversed phase or ion-exchange HPLC, or any other common method known to those skilled in the art.

In a preferred embodiment of the present invention, said removing said DNB-moiety to generate said phosphate monoester of said oligonucleotide is effected by UV irradiation, typically and preferably by UV irradiation at 365 nm, and further preferably by UV irradiation at 365 nm for 5 to 30 min, preferably for 5 to 15 min, again further preferably by UV irradiation at 365 nm for 5 to 10 min.

The present invention is further described by way of specific examples which are for illustrative purposes only and are not intended to limit the invention in any manner.

### EXAMPLES

Synthesis of some of the compounds of formula (I) is exemplified below. The following examples further illustrate the present invention, but should not be construed in any way as to limit its scope.

### EXAMPLE 1

### Synthesis of bis(2-nitrophenyl)methyl-(2-cyanoethyl)-diisopropylphosphoramidite (Ia)

A preferred compound of formula **(I)** of the present invention, phosphoramidite **(Ia)**, can be prepared in two synthetic steps with inexpensive reagents. Reaction yields were not optimized and alternative synthetic routes can be probably found.

1-lithium-2-nitrobenzene is first generated by halogen-metal exchange with phenyl lithium at low temperature and subsequently reacted with 2-nitrobenzaldehyde to afford intermediate **4**. Intermediate **4** is reacted with 2-cyanoethyl-N,N'-diisopropylchlorophosphoramidite in presence of triethylamine to yield the desired phosphoramidite **(Ia)**.

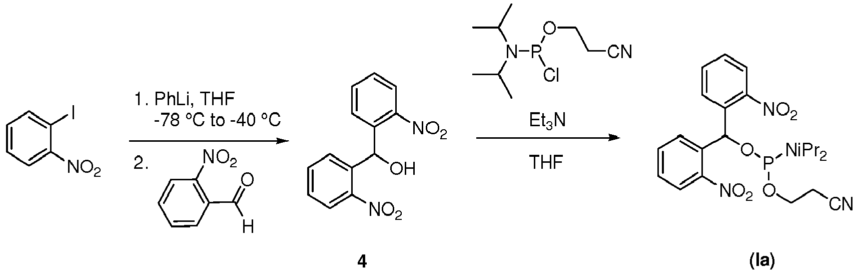

To a solution of 1-iodo-2-nitrobenzene (642 mg, 2 mmol) in THF (10 mL) at -78°C was added dropwise a 1.8M solution of phenyl lithium (1 mL, 1.8 mmol). After 30 min stirring at -78°C, the solution was warmed up to -40°C and stirred for additional 30 min. The mixture was cooled down to -78°C and 2-nitrobenzaldehyde (201 mg, 1.33 mmol) was added. After 30 min stirring at -78°C, the solution was warmed up to -40°C and stirred for additional 30 min. Water was added (1mL) and the solution was slowly warmed up to room temperature. The solution was poured into water (30 mL) and extracted three times with ethyl acetate (60 mL in total). The combined organic phase was dried over sodium sulfate, filtrated and purified by silica gel flash column chromatography (elution up to 30% EtOAc/hexane) to afford intermediate **4** (CAS 36661-36-6, 150 mg, 0.55 mmol, 41% yield). To a solution of **4** (140 mg, 0.51 mmol) in THF (2 mL) was added successively triethylamine (0.56 mL, 4.08 mmol) and 2-cyanoethyl-N,N'-diisopropylchlorophosphoramidite (482 mg, 2.04 mmol). After 2h at room temperature, the solution was poured into an aqueous solution of 5% NaHCO3 (10 mL) and extracted three times with ethyl acetate (15 mL in total). The combined organic phase was dried over sodium sulfate, filtrated and purified on silica gel by flash column chromatography (elution up to 25% EtOAc/hexane) to afford phosphoramidite **(Ia)** (170 mg, 0.36 mmol, 70% yield). 1H NMR (CDCl3, ppm) = 8.00-7.96 (m, 2H); 7.65-7.56 (m, 4H); 7.49-7.43 (m, 2H); 7.36 (d, 1H, J = 8.1 Hz); 3.80-3.55 (m, 4H); 2.57-2.44 (m, 2H); 1.16 (d, 6H, J = 6.8 Hz); 1.08 (d, 6H, J = 6.8 Hz). 31P NMR (CDCl3, ppm) = 152.7.

### EXAMPLE 2

### Synthesis of bis(2-nitro-3-methoxyphenyl)methyl-(2-cyanoethyl)-diisopropylphosphoramidite (Ib)

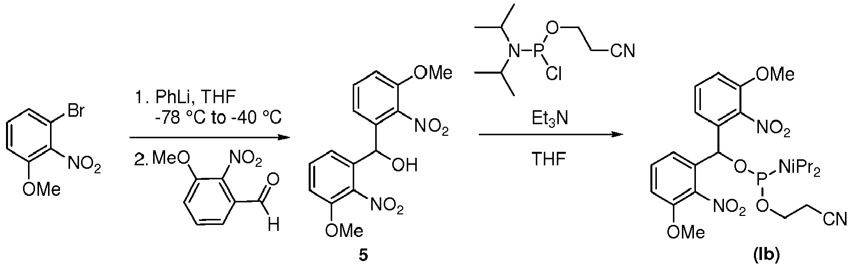

1-lithium-3-methoxy-2-nitrobenzene is first generated by halogen-metal exchange with phenyl lithium at low temperature and subsequently reacted with 3-methoxy-2-nitrobenzaldehyde to afford intermediate **5**. Intermediate **5** is reacted with 2-cyanoethyl-N,N'-diisopropylchlorophosphoramidite in presence of triethylamine to yield the desired phosphoramidite **(Ib)**.

To a solution of 1-iodo-3-methoxy-2-nitrobenzene in THF at -78°C is added dropwise a 1.8M solution of phenyl lithium. After 30 min stirring at -78°C, the solution is warmed up to -40°C and stirred for additional 30 min. The mixture is cooled down to -78°C and 3-methoxy-2-nitrobenzaldehyde is added. After 30 min stirring at -78°C, the solution is warmed up to -40°C and stirred for additional 30 min. Water is added and the solution is slowly warmed up to room temperature. The solution is poured into water and extracted three times with ethyl acetate. The combined organic phase is dried over sodium sulfate, filtrated and purified by silica gel flash column chromatography to afford intermediate **5**. To a solution of **5** in THF is added successively triethylamine and 2-cyanoethyl-N,N'-diisopropylchlorophosphoramidite. After 2h at room temperature, the solution is poured into an aqueous solution of 5% NaHCO3 and extracted three times with ethyl acetate. The combined organic phase is dried over sodium sulfate, filtrated and purified on silica gel by flash column chromatography to afford phosphoramidite **(Ib).**

### EXAMPLE 3

### Synthesis of bis(2-nitrophenyl)methyl-(2-cyanoethyl)-dimethylphosphoramidite (Ic)

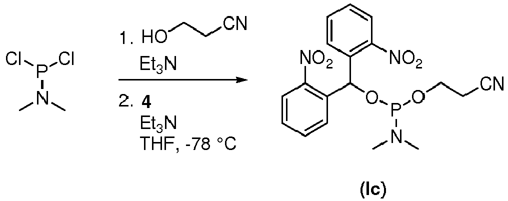

Phosphoramidite **(Ic)** is prepared by successive addition of 3-hydroxypropionitrile and intermediate **4** to dimethylphosphoramidous dichloride in presence of triethylamine.

To a solution of dimethylphosphoramidous dichloride in THF at -78°C is successively added 3-hydroxypropionitrile and triethylamine. After 1h stirring at -78°C, intermediate **4** and triethylamine are successively added to the reaction mixture which is stirred for an additional hour at -78°C. The solution is gently warmed to room temperature for 30min, poured into water and extracted three times with ethyl acetate. The combined organic phase is dried over sodium sulfate, filtrated and purified by silica gel flash column chromatography to afford phosphoramidite **(Ic)**.

### EXAMPLE 4

### Synthesis of di[bis(2-nitrophenyl)methyl]-dimethylphosphoramidite (Id)

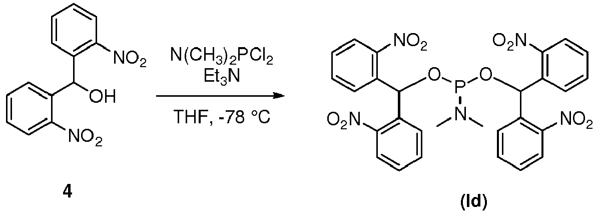

Phosphoramidite **(Id)** is prepared by addition of intermediate **4** to dimethylphosphoramidous dichloride in presence of triethylamine.

To a solution of dimethylphosphoramidous dichloride in THF at -78°C is successively added intermediate **4** and triethylamine. After 1h stirring at -78°C, the solution is warmed to room temperature and stirred for an additional 30min, poured into water and extracted three times with ethyl acetate. The combined organic phase is dried over sodium sulfate, filtrated and purified by silica gel flash column chromatography to afford phosphoramidite **(Id)**.

### EXAMPLE 5

### Syntheses of 5'-phosphate oligonucleotides

This example describes the synthesis of 5'-phosphate oligonucleotides in accordance with the present invention using the compound of formula **(Ia)**. In particular, this example describes the synthesis of several 5'-phosphate oligonucleotides of 4, 54, 60, 61, 66, 67, 68, 80 and 96 nucleotide long RNAs with a phosphate group on the terminal 5' position. The sequences of the synthesized RNAs are listed in the following, wherein, as indicated, the synthesized sequences in accordance with the present invention comprises said listed RNAs each having a phosphate group on the terminal 5' position:
5'-AUGC-3'

5’-
UGAGGUAGUAGGUUGUAUAGUUUAGAAUAACAUCACUGGAGAUAACUGUACAAC
CUCCUAG-3’ (SEQ ID NO:1)

5’-
AUAGAUUUUGGAGCAGGGAGAUGAAAUAGGAGCUUGCUCUUCCACUCCACGCG
UCGACCUGGUAUUGCAGUACCUCCAGGAAUGGGGCUCCCUCU-3’ (SEQ ID NO:2).

5’-
UGAAAAGGUAGAUAGAACAGGUCUUGUUUGCAAAAUAAAUUCAAGACCUACUUA
UCUACCAACAGCA-3’ (SEQ ID NO:3)

5’-
CCCAAUACCCCGCCAUGACGACUUGAAAUAUAGUCGGCAUUGGCAAUUUUUGACA
GUCUCUACGGAGA-3’ (SEQ ID NO:4)

5’-
GCAUGAAAUGUGACUAAUGUCUCUGUGUAAUCAGGACUUGCAACACCCUGAUUG
CUCCUAUCUGAU-3’ (SEQ ID NO:5)

5’-
AAACGCCAUUAUCACACUAAAUAGCUACUGCUAGGCAAUCCUUCCCUCGAUAAA-
3’ (SEQ ID NO:6)

5’-
UGAGGUAGUAGGUUGUAUAGUUUAGAAUUACAUCAAGGGAGAUAACUGUACAGC
CUCCUAGCUUUCC-3’ (SEQ ID NO:7)

5’-
CAAAGUGCUCAUAGUGCAGGUAGUUUUGGCAUGACUCUACUGUAGUAUGGGCAC
UUCCAG-3’ (SEQ ID NO:8)

5’-
ACCCCUGCGAUUUCCCCAAAUGUGGGAAACUCGACUGCAUAAUUUGUGGUAGUG
GGGGACUGCGUUCGCGCUUUCCCCUG-3’ (SEQ ID NO:9)

5’-
AUAGAUUUUGGAGCAGGGAGAUGAAAUAGGAGCUUGCUCUUCCACUCCACGCG
UCGACCUGGUAUUGCAGUACCUCCAGGAAUGGGGCUCCCUCU-3’ (SEQ ID NO:10)

The RNA 5'-phosphate oligonucleotide of 5'-AUGC-3'and of SEQ ID NO:1 to SEQ ID NO:10 were synthesized using an MM12 synthesizer from Bio Automation Inc on a 50-nmol scale on 1000 Å UnyLinker CPG from ChemGenes. Standard phosphoramidite solid-phase synthesis conditions were used for the synthesis. Dimethoxytrityl cleavage was accomplished with a 0.03M dichloroacetic acid in dichloromethane. 2'-TBDMS phosphoramidites were prepared as 0.1 M solutions in dry acetonitrile. 5-(Benzylthio)-1H-tetrazole (0.24 M in ACN, Carbosynth) was used to activate the phosphoramidites for coupling. Phosphoramidite coupling times were 2 x 60 s for 2'-OTBDMS amidites used as a 0.1M solution in acetonitrile and 2 x 180 s for phosphoramidite Ia (in the last coupling cycle) used as a 0.1M solution in acetonitrile. Oxidation was carried out with a 0.02 M iodine solution in a mixture composed of 70% THF, 20% pyridine and 10% of water. Capping of failed sequences was achieved with acetic anhydride in tetrahydrofuran (THF) and 16% N-methylimidazole in THF. Oligonucleotide cleavage from the support as well as nucleobase and phosphate deprotection was carried out by treatment with concentrated ammonia (25%) and concentrated aqueous methylamine (40%) at 30°C for 6 hours. The solution was filtered and the remaining RNA was washed away from the solid support with 3 x 100 µL H2O/EtOH (1:1). To the solution was added 20 µL of 1M solution of Tris-base and it was evaporated to dryness. Desilylation was carried out by treatment with 130 µL of a mixture of NMP (60 µL), TEA (30 µL) and TEA.3HF (40 µL) at 70 °C for 90 min. The reaction was quenched with trimethylethoxysilane (160 µL) for 20 min at room temperature on an Eppendorf shaker. Diethylether (1 mL) was added, the mixture was vortexed and centrifuged at 4 °C for 2 min. The supernatant was taken off and the precipitate was washed twice with 1 mL diethylether and dissolved in water. DNB-protected 5'-phosphate oligonucleotides were purified by reverse phase HPLC from Agilent equipped with a reverse phase column XBridge OST C18 from Waters allowing their separation from failure sequences. DNB-protected 5'-phosphate oligonucleotides were dried, dissolved in water, transferred to a transparent 96 well plate and irradiated for 10 min at 365nm in a Vilber Lourmat Bio-link BLX Crosslinker (5x 8 watts) inducing the deprotection of the DNB group. Final purification by reverse phase HPLC yielded the 5'-phosphate oligonucleotides. Analysis of the oligonucleotides by HPLC-MS analysis on an Agilent 1200/6130 system equipped with a Waters Acquity OST C-18 column confirmed the mass of the oligonucleotide and its high purity.

## Claims

1. A compound of formula **(I)** 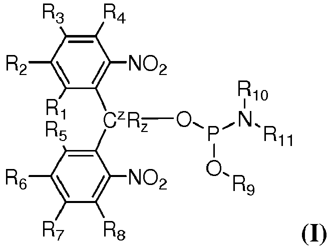 wherein
R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-; or independently thereof R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³ - independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy;
R₉ is C₁-C₉alkyl optionally substituted with cyano, nitro, halogen, -NHC(O)C₁-C₃alkyl, - NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl.

2. The compound of claim 1, wherein the 4,4'-dinitrobenzhydryl-moiety (DNB-moiety) of said compound of formula **(I)** is selected from 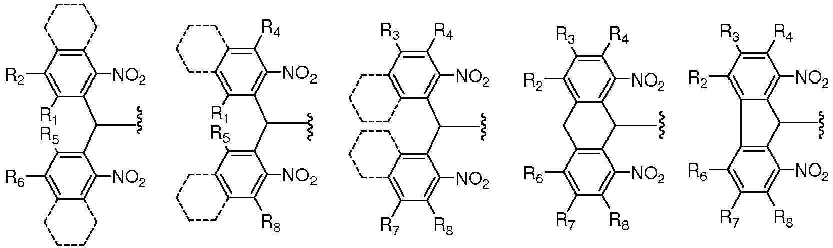 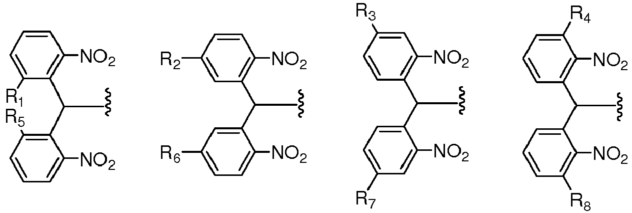
wherein R₁, R₂, R₃, R₄, R₅, R₆, R₇, and R₈ are independently of each other hydrogen, fluorine, chlorine, bromine, nitro, cyano, C₁-C₄alkyl, C₁-C₂alkoxy, C₁-C₂haloalkyl, C₁-C₂haloalkoxy; and
wherein the dotted lines represent independently of each other said bivalent residue - R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue - R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl;
and wherein the wavy line indicates the attachment to the oxygen in said compound of formula (I).

3. The compound of claim 1 or claim 2, wherein the 4,4'-dinitrobenzhydryl-moiety (DNB-moiety) of said compound of formula **(I)** is selected from 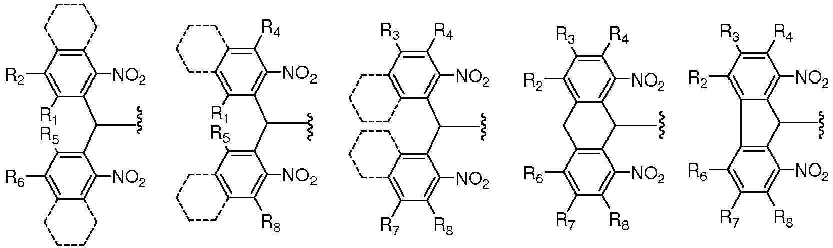 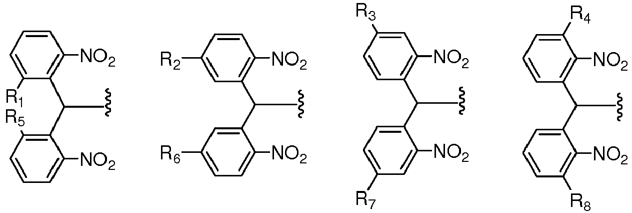
wherein R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃; and
wherein the dotted lines represent independently of each other said bivalent residue - R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue - R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl; wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine; and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl;
and wherein the wavy line indicates the attachment to the oxygen in said compound of formula (I).

4. The compound according to any one of claims 1 to 3, wherein the 4,4'-dinitrobenzhydryl-moiety (DNB-moiety) of said compound of formula **(I)** is selected from 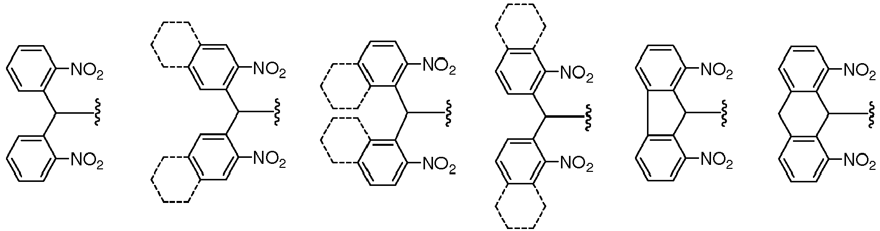 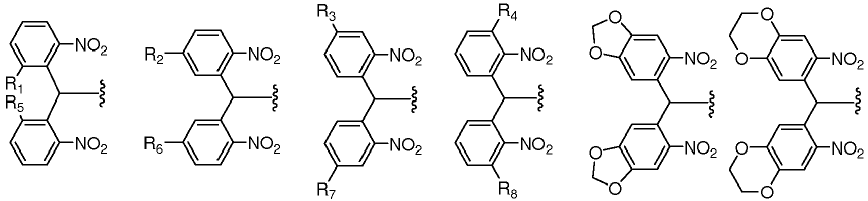
wherein R₁, R₂, R₃, R₄, R₅, R₆, R₇, and R₈ are independently of each other hydrogen, fluorine, chlorine, bromine, nitro, cyano, C₁-C₄alkyl, C₁-C₂alkoxy, C₁-C₂haloalkyl, C₁-C₂haloalkoxy; and
wherein the dotted lines represent independently of each other said bivalent residue - R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue - R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl;
and wherein the wavy line indicates the attachment to the oxygen in said compound of formula (I).

5. The compound according to any one of the claims 1 to 4, wherein the 4,4'-dinitrobenzhydryl-moiety (DNB-moiety) of said compound of formula **(I)** is selected from 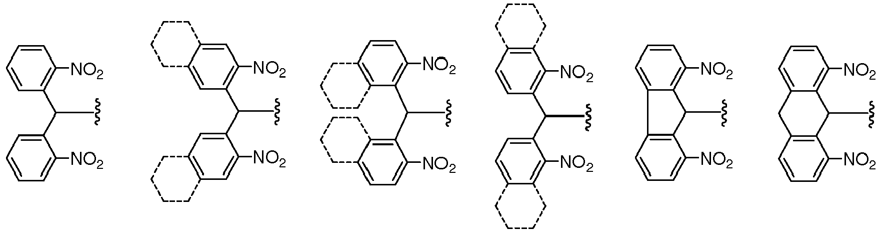 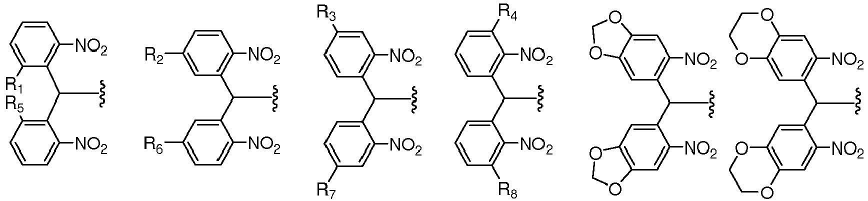
wherein said R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃;
wherein the dotted lines in said formula represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue-R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl, wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl;
and wherein the wavy line indicates the attachment to the oxygen in said compound of formula (I).

6. The compound according to any one of the claims 1 to 5, wherein R₉ is C₁-C₃alkyl optionally substituted with cyano, chlorine, fluorine and bromine; aryl, C₁-C₃alkylenearyl, C₁-C₃alkylenediaryl, independently of each other optionally substituted with cyano, nitro, chlorine, fluorine, bromine, C₁-C₂alkoxy, C₁haloalkyl.

7. The compound according to any one of the claims 1 to 6, wherein -NR₁₀R₁₁ is selected from

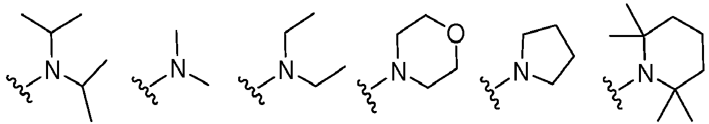

8. The compound according to any one of the claims 1 to 7, wherein the compound is selected from 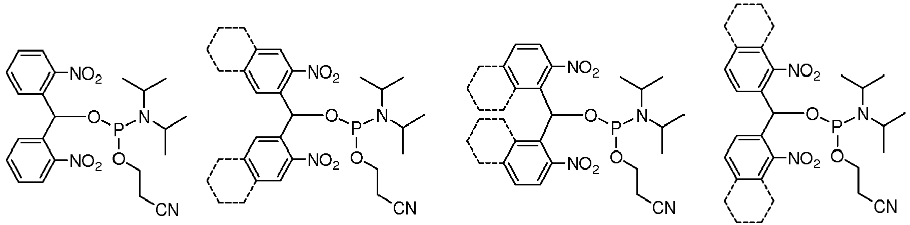 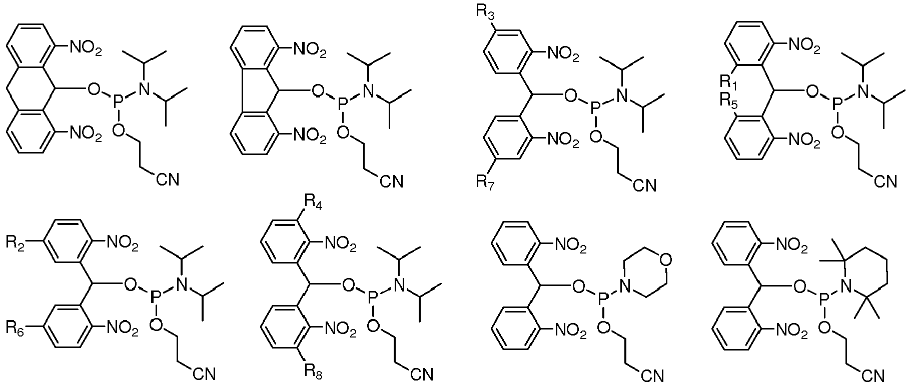 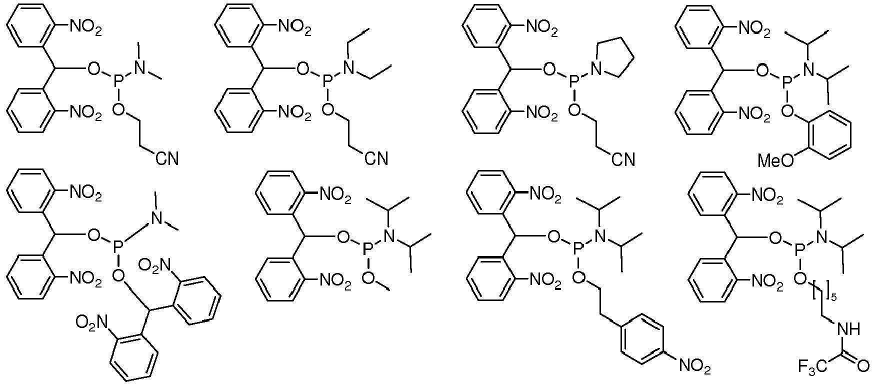
wherein said R₁, R₂, R₃, R₄, R₅, R₆, R₇, and R₈ are independently of each other hydrogen, fluorine, chlorine, bromine, nitro, cyano, methyl, ethyl, iso-propyl, tert-butyl, OCH₃, CF₃, OCF₃;
wherein the dotted lines in said formula represent independently of each other said bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof
wherein the dotted lines represent independently of each other said bivalent residue-R¹²R¹³, wherein each of said bivalent residue -R¹²R¹³- independently at each occurrence together with the vicinal C atoms to which they are attached independently of each other form a 5-7-membered heterocyclic ring or a 5-7-membered hetero-aromatic ring, each independently optionally substituted with halogen, nitro, cyano, C₁-C₂alkyl C₁-C₂alkoxy, C₁-C₂haloalkyl, wherein said 5-7-membered heterocyclic ring is independently at each occurrence selected from pyrrolidine, pyrazolidine, imidazolidine, piperidine, piperazine, dioxane, dioxolane, tetrahydrofurane, morpholine, oxazolidine and isooxazolidine, and wherein said 5-7-membered hetero-aromatic ring is independently at each occurrence selected from pyrrolidine, piperidine, piperazine, morpholine, pyridine, pyrimidine, pyrazine, pyridazine, pyrrole, pyrazole, imidazole, triazole, isoxazole, oxazole, isothiazole, thiazole, tetrazole, furane, and thiophenyl.

9. The compound according to any one of the claims 1 to 8, wherein said R₁ is equal to R₅, said R₂ is equal to R₆, said R₃ is equal to R₇, or said R₄ is equal to R₈.

10. The compound according to any one of the claims 1 to 9, wherein said R₁ is equal to R₅, said R₂ is equal to R₆, said R₃ is equal to R₇, and said R₄ is equal to R₈.

11. The compound according to any one of the claims 1 to 10, wherein the selection of R₁, R₂, R₃, R₄, R₅, R₆, R₇, R₈ and R_{z} together is such that the carbon atom C^{Z} is not an asymmetric carbon atom.

12. The compound according to any one of the claims 1 to 11, wherein the compound is selected from the compound of formula **(Ia), (Ib), (Ic)** and **(Id)**

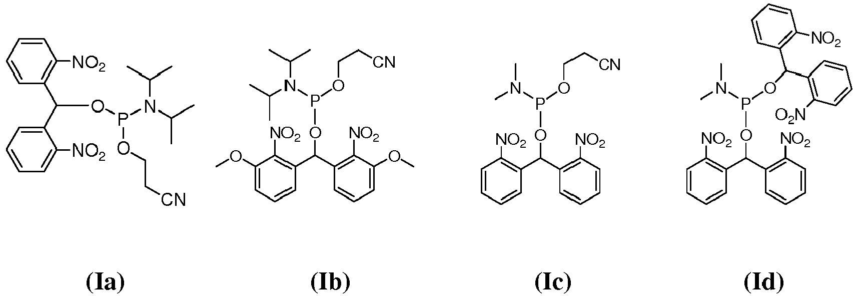

13. The compound according to any one of the claims 1 to 12, wherein the compound is the compound of formula **(Ia)**

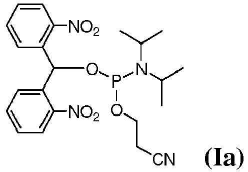

14. A method of converting a terminal hydroxyl group of an oligonucleotide, preferably of converting a terminal 5'-hydroxyl group of an oligonucleotide, into a phosphate monoester, comprising of the steps of:
a) reacting a compound of formula (I) with said terminal hydroxyl group of an oligonucleotide, 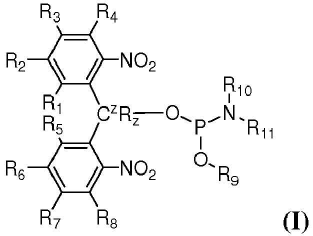
wherein R₁, R₂, R₃, R₄, R₅, R₆, R₇, and R₈ are independently of each other hydrogen, halogen, nitro, cyano, C₁-C₆alkyl, C₃-C₆cycloalkyl, C₁-C₄alkoxy, C₁-C₄haloalkyl, C₁-C₄haloalkoxy, each independently of each other optionally substituted with cyano, nitro, halogen; or independently thereof
R₁ and R₅ represent together a bond or form together -CH₂-; or independently thereof R₁ and R₂, R₂ and R₃, R₃ and R₄, R₅ and R₆, R₆ and R₇, R₇ and R₈ form together independently of each other a bivalent residue -R¹²R¹³-, wherein each of said bivalent residue -R¹²R¹³- independently at each occurrence is C₃-C₅alkylene, C₃-C₅alkenylene or C₃-C₅alkdienylene, each independently optionally substituted with halogen, nitro, cyano, C₁-C₂alkyl C₁-C₂alkoxy, C₁-C₂haloalkyl; or independently thereof wherein each of said bivalent residue -R¹²R¹³- independently at each occurrence together with the vicinal C atoms to which they are attached independently of each other form a heterocyclic ring or a hetero-aromatic ring, each independently optionally substituted with halogen, nitro, cyano, C₁-C₂alkyl C₁-C₂alkoxy, C₁-C₂haloalkyl;
R_{z} is H, C₁-C₆alkyl optionally substituted with halogen, C₁-C₃alkoxy, or R_{z} is phenyl optionally substituted with halogen, C₁-C₃alkyl, C₁-C₃alkoxy;
R₉ is C₁-C₉alkyl optionally substituted with cyano, nitro, halogen, -NHC(O)C₁-C₃alkyl, -NHC(O)C₁-C₃haloalkyl, C₁-C₃alkylsulfonyl; aryl, C₁-C₆alkylenearyl, C₁-C₆alkylenediaryl independently of each other optionally substituted with cyano, nitro, halogen, C₁-C₄alkoxy, C₁-C₄haloalkyl, C₁-C₄haloalkoxy, -NHC(O)C₁-C₃alkyl, NHC(O)C₁-C₃haloalkyl, C₁-C₃alkylsulfonyl;
R₁₀ and R₁₁ are independently of each other C₁-C₉alkyl optionally substituted with cyano, nitro, halogen, C₂-C₆alkenyl, C₃-C₆cycloalkyl, C₁-C₃alkoxy, phenyl optionally substituted with cyano, nitro, halogen, C₁-C₃ alkyl, C₁-C₃alkoxy; or together with the nitrogen atom to which they are attached form a heterocyclic ring, wherein preferably said heterocyclic ring is selected from pyrollidinyl, piperidinyl, morpholinyl, piperazinyl and homopiperazine, wherein said heterocyclic ring is optionally substituted with C₁-C₃ alkyl;
b) oxidizing the P atom of compound (I) of the resulting product of a) and removing said R₉ residue to generate a phosphate diester of said oligonucleotide comprising said DNB-moiety;
c) purifying said phosphate diester of said oligonucleotide of b);
d) removing said DNB-moiety comprised by said phosphate diester of said oligonucleotide to generate said phosphate monoester of said oligonucleotide.

15. The method of claim 14, wherein said compound of formula (I) is defined as in any one of the claims 1 to 13.


SEQUENCE LISTING

<110>  ETH Zurich

<120>  NOVEL PHOSPHORYLATION REAGENTS AND USES THEREOF

<130>  P5050EP00

<160>  10

<170>  PatentIn version 3.5

<210>  1
<211>  61
<212>  RNA
<213>  artificial sequence

<220>
<223>  synthetic RNA oligonucleotide

<400>  1
ugagguagua gguuguauag uuuagaauaa caucacugga gauaacugua caaccuccua      60

g                                                                      61


<210>  2
<211>  96
<212>  RNA
<213>  artificial sequence

<220>
<223>  synthetic RNA oligonucleotide

<400>  2
auagauuuuu ggagcaggga gaugaaauag gagcuugcuc uuccacucca cgcgucgacc      60

ugguauugca guaccuccag gaauggggcu cccucu                                96


<210>  3
<211>  67
<212>  RNA
<213>  artificial sequence

<220>
<223>  synthetic RNA oligonucleotide

<400>  3
ugaaaaggua gauagaacag gucuuguuug caaaauaaau ucaagaccua cuuaucuacc      60

aacagca                                                                67


<210>  4
<211>  68
<212>  RNA
<213>  artificial sequence

<220>
<223>  synthetic RNA oligonucleotide

<400>  4

cccaauaccc cgccaugacg acuugaaaua uagucggcau uggcaauuuu ugacagucuc 60

uacggaga 68


<210> 5
<211> 66
<212> RNA
<213> artificial sequence

<220>
<223> synthetic RNA oligonucleotide

<400> 5
gcaugaaaug ugacuaaugu cucuguguaa ucaggacuug caacacccug auugcuccua 60

ucugau 66


<210> 6
<211> 54
<212> RNA
<213> artificial sequence

<220>
<223> synthetic RNA oligonucleotide

<400> 6
aaacgccauu aucacacuaa auagcuacug cuaggcaauc cuucccucga uaaa 54


<210> 7
<211> 67
<212> RNA
<213> artificial sequence

<220>
<223> synthetic RNA oligonucleotide

<400> 7
ugagguagua gguuguauag uuuagaauua caucaaggga gauaacugua cagccuccua 60

gcuuucc 67


<210> 8
<211> 60
<212> RNA
<213> artificial sequence

<220>
<223> synthetic RNA oligonucleotide

<400> 8
caaagugcuc auagugcagg uaguuuuggc augacucuac uguaguaugg gcacuuccag 60


<210> 9
<211> 80
<212> RNA
<213> artificial sequence

<220>

<223>  synthetic RNA oligonucleotide

<400>  9
accccugcga uuuccccaaa ugugggaaac ucgacugcau aauuuguggu agugggggac      60

ugcguucgcg cuuuccccug                                                  80



<210>  10
<211>  96
<212>  RNA
<213>  artificial sequence

<220>
<223>  synthetic RNA oligonucleotide

<400>  10
auagauuuuu ggagcaggga gaugaaauag gagcuugcuc uuccacucca cgcgucgacc      60

ugguauugca guaccuccag gaauggggcu cccucu                                 96